# EUROPEAN PATENT SPECIFICATION

(11) **EP 0 543 076 B1**
(45) Date of publication and mention of the grant of the patent: **23.09.1998**
(21) Application number: 92101636.6
(22) Date of filing: 31.01.1992
(51) Int. Cl.: A61K 38/55, C07K 2/00

(54) **Wheat alpha-amylase inhibitor for the treatment of diabetes**
Weizen Alpha-Amylase-Inhibitor zur Behandlung von Diabetes
Alpha-amylase inhibiteur de blé pour le traitement du diabète

(30) Priority: 18.11.1991 JP 301684/91
(43) Date of publication of application: 26.05.1993
(73) Proprietor: YAKURIGAKU CHUO KENKYUSHO, Tokyo (JP)
(72) Inventor: Takahashi, Hidehiko, Setagaya-ku, Tokyo (JP)
(74) Representative: Bühling, Gerhard, Dipl.-Chem.

(56) References cited:
- EP-A- 0 297 834
- EP-A- 0 372 522
- EP-A- 0 372 523
- US-A- 3 950 319
- JAPANESE JOURNAL OF PHARMACOLOGY vol. 55, no. S1 , 1991 , KYOTO,JAPAN page 125P DOI ET AL 'ATTENUATED HYPERGLYCEMIA AND ELEVATED 3-HYDROXYBUTYRIC ACID IN RESPONSE TO ALPHA-AMYLASE INHIBITOR IN RATS'
- DATABASE WPI Section Ch, Week 8637, Derwent Publications Ltd., London, GB; Class B04, AN 86-242361 'AMYLASE INHIBITING MATERIAL EXTRACTED FROM GLUTEN-USED IN TREATMENT OF E.G. DIABETES' & JP-A-61 171 431 (TOYOBO KK) 2 August 1986
- CHEMICAL ABSTRACTS, vol. 99, no. 11, 12 September 1983, Columbus, Ohio, US; abstract no. 84271u, WESELAKE ET AL 'AN ENDOGENOUS ALPHA-AMYLASE INHIBITOR IN BARLEY KERNELS' page 241 ;column 2 ; & PLANT PHYSIOL. vol. 72, no. 3 , 1983 pages 809 - 812

## Description

### BACKGROUND OF THE INVENTION

### FIELD OF THE INVENTION

The present invention relates to a composition for the treatment of diabetes comprising as an active ingredient a partially purified, novel α-amylase inhibitor, as an effective auxiliary means for diet therapy of diabetes from which more than 10% of Japanese over 40 years old are suffering.

### PRIOR ART

Diet therapy (calorie control) and blood sugar depressants have been applied to the treatment of insulin-independent diabetes (diabetes mellitus type II).

It is known that blood sugar depressants have various side effects and a danger of hypoglycemia is not negligible, either. Diet therapy comprises giving a low calorie diet, especially a super low calorie diet as its main thereby to render blood sugar level and body weight normal. However, such a therapy is unexpectedly unsuccessful because of strong hunger sensation, impossible continuation over a long period of time, rebound phenomena by interruption, etc.

For the reasons above, investigations have been focused on control of glucose absorption in the small intestine. Along with this line, administration of α-glucosidase inhibitors, inter alia, acarbose was successful. It has been confirmed that this digestive enzyme inhibitor retards absorption of glucose after meals to cause reduction in secretion of insulin. However, it is yet unclear if the inhibitor is effective for the treatment of diabetes. In addition, many side effects are noted with the inhibitor [M. Toeller, Modulation of intestinal glucose absorption postponement of glucose absorption by α-glucosidase inhibitors in Pharmacology of Diabetes, edited by C.E. Mogensen and E. Standl, 93-112, Walter de Gruyter, Berlin, 1991; L. Mueller and W. Pul, Pharmacology of α-glucosidase inhibitors in structure and function of the small intestine, edited by W.F. Caspary, 281-300, Exerpta Medica, Amsterdam, 1987; U.R. Foelsch and B. Lembcke, The clinical use of α-glucosidase inhibitors in structure and function of the small intestine, edited by W.F. Caspary, 301-318, Excerpta Medica, Amsterdam, 1987].

On the other hand, investigations have also been made on application of starch digestive enzyme α-amylase inhibitors to the treatment of diabetes but are not successful yet [B. Lembcke, Control of absorption: delaying absorption as a therapeutic principle in structure and function of the small intestine, edited by W.E. Caspary, 263-280, Excerpta Medica, Amsterdam, 1987].

The present invention aims at providing a pharmaceutical preparation comprising a partially purified α-amylase inhibitor (of wheat flour origin) having a high activity, which exhibits an excellent therapeutic effect without being accompanied by side effects such as meteorism, diarrhea, etc. caused by an α-glucosidase inhibitor. The present invention also aims at treating obesity which causes not only diabetes (type II) but also dangerous complications. The α-glucosidase inhibitor fails to reduce body weight.

Glucose absorption in the small intestine can be controlled by inhibiting digestion of starch at various stages. However, it is theoretically deduced that inhibition on a starch level, i.e., inhibition of α-amylase would cause abnormal fermentation in the colon (meterorism) or diarrhea (osmotic diarrhea) with far less frequency, than in inhibition of α-glucosidase. Energy sources absorbed by decomposition of bacterial flora in the colon is less so that it is expected that reduction in body weight by α-amylase inhibitor would be larger than in the case of α-glucosidase inhibitor. It is thus expected that α-amylase inhibitor would affect fat metabolism more.

In all aspects, α-amylase inhibitor is more advantageous than α-glucosidase inhibitor.

### SUMMARY OF THE INVENTION

According to the present invention, there is provided a composition for the treatment of diabetes comprising as an active ingredient the purified product (hereinafter referred to as Deobesitogen) obtained by subjecting an α-amylase inhibitor of flour origin to column chromatography to remove a part of sugar and inorganic matters from the α-amylase inhibitor and then drying. The aforesaid problems have thus been solved.

### BRIEF DESCRIPTION OF THE DRAWINGS

Fig. 1 is a graph showing disappearance of 30HBA in blood by administration of Deobesitogen according to the present invention.

Fig. 2 is a graph showing effects by administration of Deobesitogen tablet of the present invention to a patient with borderline type diabetes.

Fig. 3 is a graph showing effects by administration of Deobesitogen tablet of the present invention to another patient with borderline type diabetes as in Fig. 2.

Fig. 4 shows the activity of reducing body weight by administration of Deobesitogen (anti-obesity effect), wherein DM, BDM, OH and n indicate patients with diabetes mellitus, patients with broderline type diabetes mellitius, patients with hyperinsulin obesity and the number of cases, respectively.

Fig. 5 shows the activity of improving blood sugar level in hunger when Deobesitogen was administered.

Fig. 6 shows the activity of improving HbA₁c of Deobesitogen.

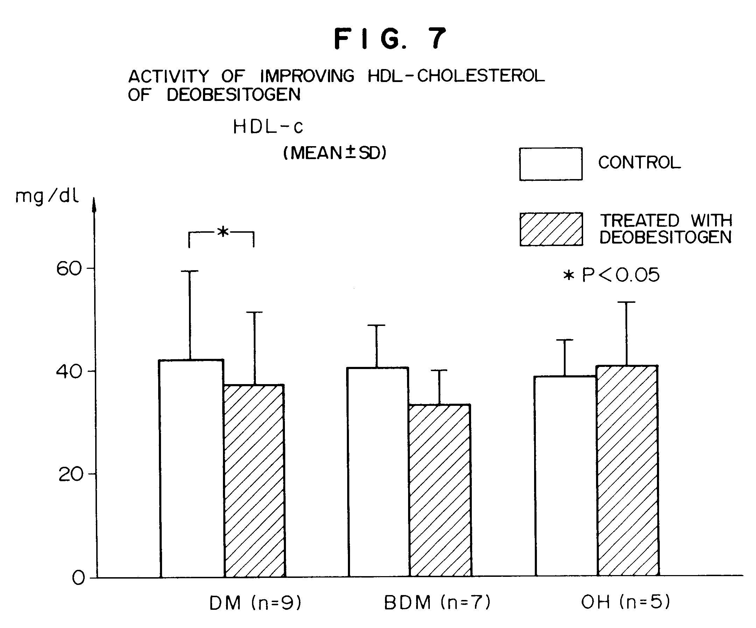

Fig. 7 shows the activity of improving HDL-cholesterol of Deobesitogen.

### DETAILED DESCRIPTION OF THE PREFERRED EMBODIMENTS

As sources for the α-amylase inhibitor, a wheat albumen is preferred since its inhibitor content is the highest and its hemoglutinin (lectin) content is the lowest among cereals and beans and hence, it is possible to concentrate the wheat albumen. It is thus expected to obtain preparations having a high activity at low costs.

In Japanese Patent Application Laid-Open No. 2-157297, the present inventor revealed the activity of reducing body weight using a substance which inhibits an amylase activity and is obtained by partially purifying an α-amylase inhibitor by chromatography. During the course of investigations, the inventor found that the partially purified α-amylase inhibitor affected blood sugar level and neutral fat in blood, etc. [U.R. Foelsch and B. Lembcke, The clinical use of α-glucosidase inhibitors in structure and function of the small intestine, edited by W.F. Caspary, 301-318, Excerpta medica, Amsterdam, 1987].

In assistance of clinicians, the inventor has made extensive pursuits therapeutic effects of Deobesitogen on patients with diabetes and confirmed remarkable activities that Deobesitogen has an activity of lowering blood sugar level of patient with diabetes to normal level but does not reduce at all to a level lower than normal and reduces neutral fat level only when the level is higher than normal. The present invention has thus been accomplished.

In the present invention, the removal of sugar by column chromatography has greatly contributed to the improved activity of α-amylase inhibitory activity (αAI level) of the extract of wheat with water.

### Preparation Example

Sephadex G75® was dipped in a phosphoric acid buffer (pH 6.8, ionic intensity 0.05) for 24 hours, to allow to be enough swollen. A recycling chromatography column in a total volume of 10 ℓ was filled with the thus-swollen Sephadex G75®.

A supernatant liquid of an aqueous extract of wheat flour was heated to modify insoluble protein, and then the resulting modified protein was removed. From the thus-obtained solution, low molecular compounds were further filtered through a ultra-filter membrane. The filtrate was concentrated, and the concentrate was subjected to a drying treatment to obtain a solid substance as a sample.

The resulting sample was treated as follows:

100 ml of 1% sample solution was upwardly eluted out from the lower end of the column and recycled in thereby having collected a fraction of protein having the strongest amylase activity the column, while recording the absorption of protein in the eluting solution by means of UV ray detector flow monitor, thereby having collected a protein fraction having molecular weight of about 10,000 - 50,000, which is strongest in α-amylase inhibitor activity.

### Example 1 Effect of Deobesitogen on rats with experimental diabetes

Steptozocine (STZ) was administered to Wister strain male rats of 7 weeks age. The thus prepared rats with diabetes were fed with powder feed mainly composed of α starch. Deobesitogen was administered to animal in a dose of 10 mg/kg for 4 weeks, whereby body weight, blood sugar level, HbA₁c, lipid, etc. were traced every 2 other weeks. With respect to peripheral nerve disorders as complications, nerve excitation conductive rate at the tail was determined.

By administration of Deobesitogen, increase in body weight of rats with diabetes was prevented and blood sugar level was lowered to normal level (blood sugar level in healthy control group) four weeks after.

HbA₁c also kept decreasing. A decrease was noted with total cholesterol, neutral fats and phospholipids in the group treated with Deobesitogen for 4 weeks.

Also in nerve excitation conductive rate, a significant improvement was noted between the group treated for 4 weeks and the diabetes group.

**Table 1**

| | Body weight | Blood sugar level | HbA₁ | HbA₁c | Total cholesterol | TG | (PL) |
|---|---|---|---|---|---|---|---|
| Normal rat | 420±42 | 127±27 | 3.6±1.3 | 2.20±0.3 | 84±19 | 88±26 | 131±25 |
| STZ rat | 313±62 | 171±53 | 3.7±0.7 | 2.4±0.2 | 92±6 | 167±109 | 179±31 |
| Rat given with STZ + Deobesitogen | 305±34 | 131±19 | 3.0±0.6 | 1.6±1.1 | 80±9 | 101±46 | 144±18 |

### Example 2 Effect of Deobesitogen on increase of 3-hydroxybutyric acid

The run was performed using Wistar King A male mature rats weighing 280-350 g. The animals were fed with starch-rich feed (control group) and starch-rich feed containing 5% Deobesitogen (test group). Intake of feed was limited to 10 g per day and the animals were fed for consecutive 3 days.

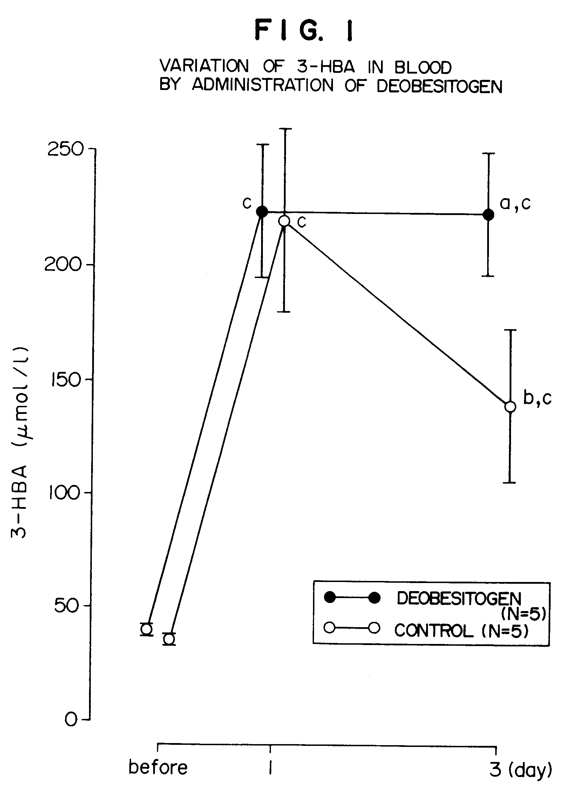

Change of 3-hydroxybutyric acid (3-HBA) with passage of time under limited feeding is shown in Fig. 1. In both groups, 3-HBA increased one day after (p 0.01). The increase of 3-HBA 3 days after was almost the same as that one day after and higher than the control group (p 0.05). In the control group, the increase of 3-HBA 3 days after was smaller than that one day after (p 0.05) but higher than that prior to administration (p 0.05) (Fig. 1).

It is considered that Deobesitogen would retard an increase of blood sugar to inhibit secretion of insulin so that fat degradation would be promoted and 3-HBA would increase. Taking into account that oversecretion of insulin accelerates obesity by fat synthesis and 3-HBA participates in alimentary depression and satiety, it is expected that deobesitogen would be usable for the patient with diabetes.

### Example 3 Influence of Deobesitogen on body weight and blood sugar level of the patient with borderline diabetes mellitius type II

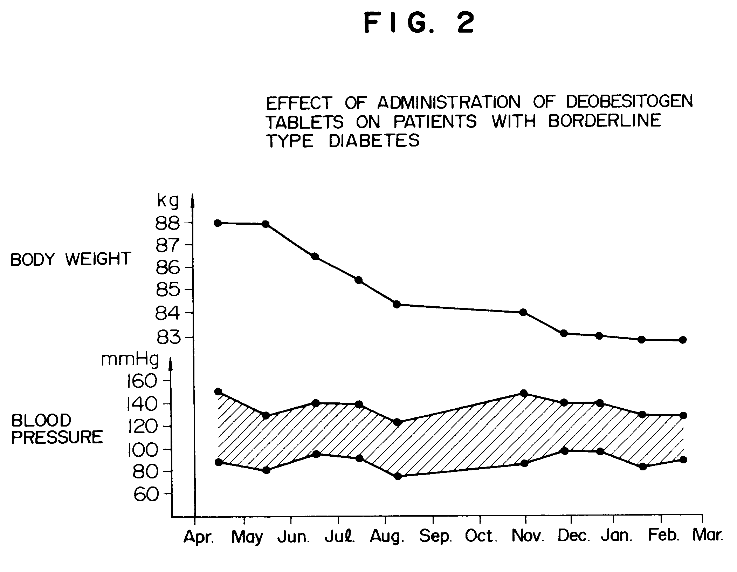

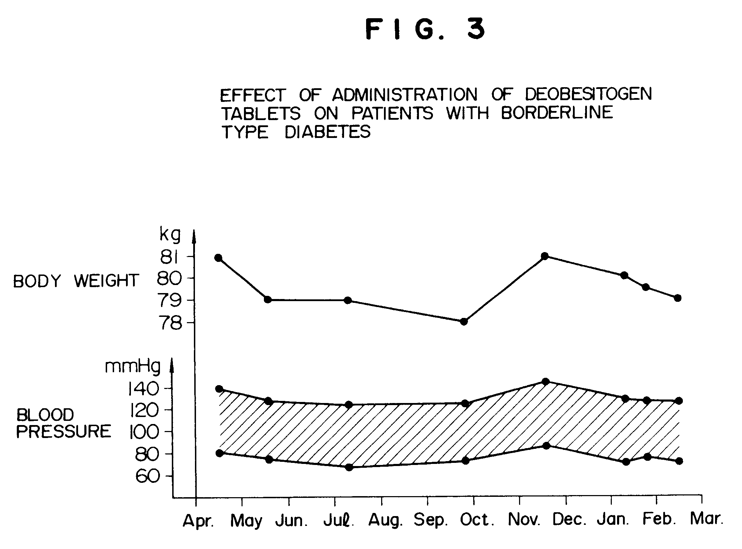

The results on two cases are shown in Figs. 2 and 3. It was noted that body weight, blood sugar level and neutral fat level in blood were improved.

### Example 4 Therapeutic effect of Deobesitogen on diabetes mellitius type II

The experiment was performed on 9 patients with diabetes, 7 patients with borderline type diabetes and 5 patients with insulin-oversecretive obesity.

Findings prior to administration of Deobesitogen were compared with findings 8 weeks after administration of Deobesitogen. The results are shown below. The patients had the same diet and life style as before but no restriction. Six Deobesitogen tablets were administered before meals.

### Effects of administration of Deobesitogen:

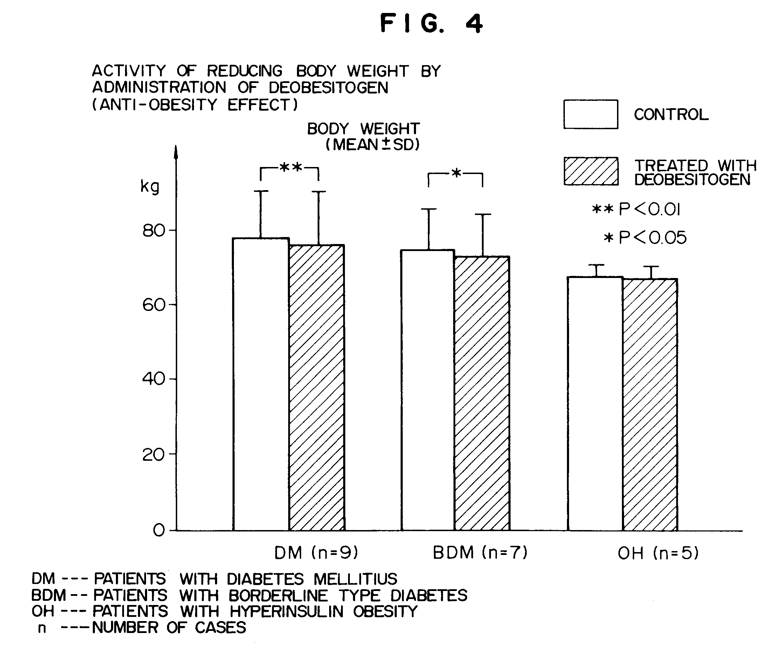

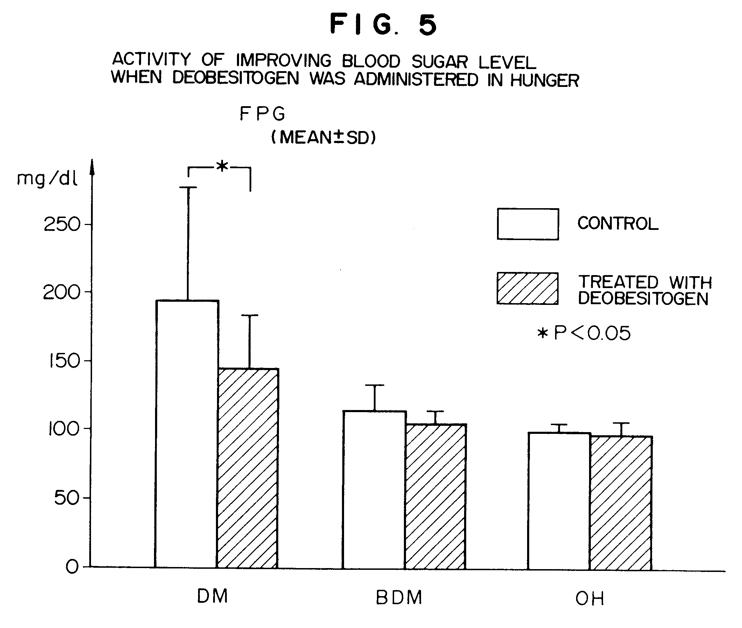

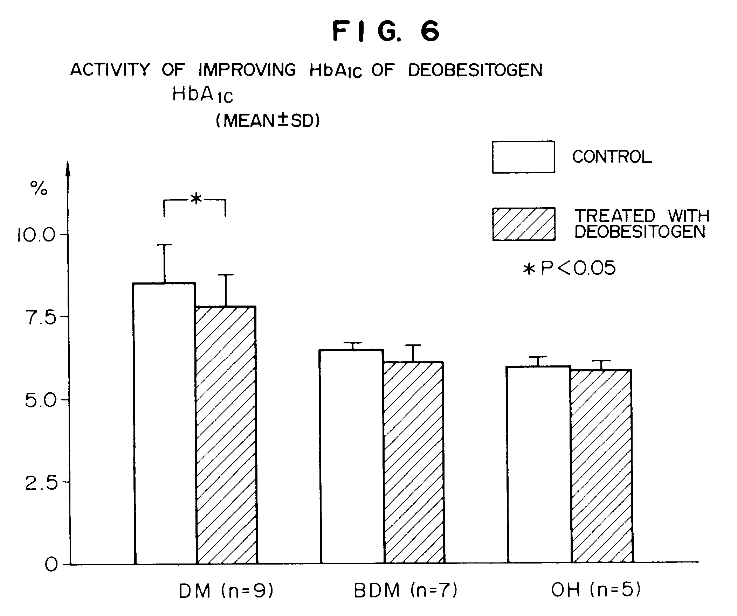

1) Body weight of the patients with diabetes and the patients with borderline type diabetes showed a significant decrease (Fig. 4).
2) FPG (fasting plasma glucose) level and HbA₁c level showed a significant decrease in the patients with diabetes (Figs. 5 and 6).
3) HDL-cholesterol level showed a significant decrease in the patients with diabetes.
4) TG level and FLDL TG level in hunger and after meals significantly decreased.

From the foregoing results, the therapeutic effects of Deobesitogen on diabetes (type II) were verified.

In all cases, side effects such as meteorism, diarrhea, etc. were noted at all.

A part of the studies was reported in the International Diabetes Association on June 26, 1991 held in Washington D.C. of America.

The present invention relates to a composition for the treatment of diabetes mellitus type II comprising Deobesitogen as an active ingredient. Deobesitogen is a partially purified α-amylase inhibitor obtained by subjecting a concentrate of the aqueous extract from wheat flour to column chromatography to remove sugar and a part of inorganic matters from the α-amylase inhibitor and then drying.

The composition of the present invention exhibits such a remarkable effect on the treatment by administering the drug to the patient with diabetes mellitus type II that does not necessitate calorie control.

## Claims

1. A composition for the treatment of diabetes comprising as an active ingredient a partially purified α-amylase inhibitor obtained from a concentrate of the aqueous extract of wheat flour by means of a ultra-filter and a gel column chromatography using Sephadex® as a carrier, and then drying, thereby collecting a protein fraction having molecular weight of about 10,000 - 50,000, which is strongest in α-amylase inhibitor activity.

2. A composition according to claim 1, wherein said partially purified α-amylase inhibitor is Deobesitogen.

## Patentansprüche

1. Zusammensetzung für die Behandlung von Diabetes, umfassend als aktiven Bestandteil einen teilweise gereinigten α-Amylase-Hemmstoff, der aus einem Konzentrat eines wäßrigen Weizenmehlextrakts durch Ultrafiltration und Gelsäulenchromatographie unter Verwendung von Sephadex® als Träger und anschließendem Trocknen erhalten wurde, wobei eine Proteinfraktion mit einem Molekulargewicht von etwa 10000 bis 50000 und der stärksten α-Amylase-Hemmaktivität gesammelt wurde.

2. Zusammensetzung nach Anspruch 1, wobei der teilweise gereinigte α-Amylase-Hemmstoff Deobesitogen ist.

## Revendications

1. Composition pour le traitement du diabète comprenant en tant que principe actif un inhibiteur de l'α-amylase partiellement purifié, obtenu à partir d'un concentré de l'extrait aqueux de farine de blé au moyen d'une ultrafiltration et d'une chromatographie sur colonne de gel en utilisant du Sephadex® en tant que support, en séchant et en recueillant ainsi une fraction de protéine ayant un poids moléculaire d'environ 10 000-50 000, qui a la plus forte activité d'inhibition de l'α-amylase.

2. Composition selon la revendication 1, dans laquelle ledit inhibiteur de l'α-amylase partiellement purifié est le Deobesitogen.
